# EUROPEAN PATENT SPECIFICATION

(11) **EP 4 335 431 B1**
(45) Date of publication and mention of the grant of the patent: **20.08.2025**
(21) Application number: 23382773.2
(22) Date of filing: 25.07.2023
(51) Int. Cl.: A61Q 11/00, A61K 8/21, A61K 36/28, A61K 45/06, A61K 8/9789, A61K 36/537, A61P 17/06, A61P 17/00, A61K 8/20, A61K 36/899, A61Q 19/00, A61K 8/23, A61K 31/05, A61K 36/38, A61K 36/16, A61K 36/258, A61K 36/185, A61K 36/23

(54) **COMPOSITION WITH MAGNESIUM SALTS**
ZUSAMMENSETZUNG VON MAGNESIUMSALZEN
COMPOSITION DES SELS DE MAGNÉSIUM

(43) Date of publication of application: 13.03.2024
(73) Proprietor: Instituto de Dermatologia Ignacio Umbert, S.L., 08017 Barcelona (ES)
(72) Inventor: UMBERT MILLET, Ignacio, 08017 BARCELONA (ES)
(74) Representative: Isern Patentes y Marcas S.L.

(56) References cited:
- WO-A1-2020/095099
- US-A1- 2008 038 219
- DATABASE GNPD [online] MINTEL; 16 May 2017 (2017-05-16), ANONYMOUS: "Green Mud Face Mask", XP093100853, retrieved from https://www.gnpd.com/sinatra/recordpage/4813273/ Database accession no. 4813273

## Description

### Technical field of the invention

The present invention relates to a composition, characterised in that it comprises at least one magnesium salt, at least one salt not comprising magnesium, at least one antioxidant, and at least one plant extract. The composition of the invention is intended for the treatment of inflammation, in particular inflammation in the skin, for example: dermatitis, atopic dermatitis, vitiligo, psoriasis or pruritus, and combinations thereof.

### Background of the invention

Skin inflammation is a problem that can be caused by various factors, such as environmental factors, allergies, irritants, medical conditions, stress, and genetics. It is a common condition characterised by redness, itching, swelling, and a burning sensation on the surface of the skin or mouth. Preventing oral inflammation is critical to maintaining oral health and avoiding oral problems such as lichen planus, an autoimmune disease that affects the lining of the mouth and can lead to painful lesions and discomfort. In addition, it is essential to take care of the skin and take measures to prevent autoimmune lesions at a cutaneous and systemic level (such as psoriasis, atopic dermatitis, itching, vitiligo and cancer prevention) and other processes.

Existing technology to treat inflammation in the skin includes creams, lotions, ointments or mouthwashes containing topical corticosteroids, antihistamines, mouthwashes with special solutions and other active ingredients. However, these treatments often have unwanted side effects and are not effective for everyone. Some medications, such as certain antibiotics, non-steroidal anti-inflammatory drugs, and hypertension medications, can also trigger or aggravate the disease in certain individuals.

In addition, the use of natural ingredients in the formulation of products to treat skin inflammation has been investigated. For example, aloe vera and calendula have been shown to have anti-inflammatory and soothing properties that can help reduce skin inflammation. However, these natural ingredients are often unstable and may lose their effectiveness in the manufacturing and/or application process.

In short, adequate prevention of skin inflammation at a systemic or local level in the mouth and skin protection are crucial measures to avoid neoplastic skin in addition to preventing autoimmune disorders in the body. Accordingly, there is an unmet need for an effective and safe composition for treating irritation that does not have unwanted side effects and that contains stable and effective natural ingredients.

Several compositions containing magnesium are known in the prior art. For example, US2008/038219 A1 discloses a topical composition consisting of carboxylic acids, chelating agents, dimethyl sulfone, and magnesium sulphate, which serves as a base for the application of medications and active ingredients. Additionally, WO 2020/095099 discloses compositions that include magnesium, various non-magnesium salts, and plant extracts. However, these compositions are designed for oral cosmetic use due to their remineralizing properties, which restore tooth enamel and reduce tooth sensitivity.

Similarly, there are cosmetic products for skin care that contain magnesium salts, variousnon-magnesium salts, plant extracts, and antioxidants, such as GIGOT's facial mask "*Instant mask mascarilla facial fango verde con alga espirulina.*" However, these products are intended for daily skin care and not for the treatment of skin inflammation or irritation.

The present invention meets this need by the formulation of an innovative composition utilising safe, natural active ingredients that have been shown to be effective in treating inflammation occurring in the skin.

### Summary of the invention

The present invention resolves the aforementioned drawbacks by means of a composition comprising at least one magnesium salt among other natural ingredients.

The first aspect of the invention relates to a topical anti-inflammatory composition for use in a treatment of skin inflammation characterised in that it comprises at least one magnesium salt, at least one salt not comprising magnesium, at least one antioxidant, and at least one plant extract, wherein the at least one salt not comprising magnesium is a mixture of sodium chloride, sodium fluoride and potassium chloride.

The combination of compounds of the composition results in an increase in anti-inflammatory efficacy. Thus, in general, the resulting formulations of the present invention allow eliminating from the composition the aggressive substances such as corticosteroids, antibiotics, enhancing, surprisingly, by a synergistic effect, the anti-inflammatory effect of the compounds of the composition at low concentrations.

### Brief description of the drawings

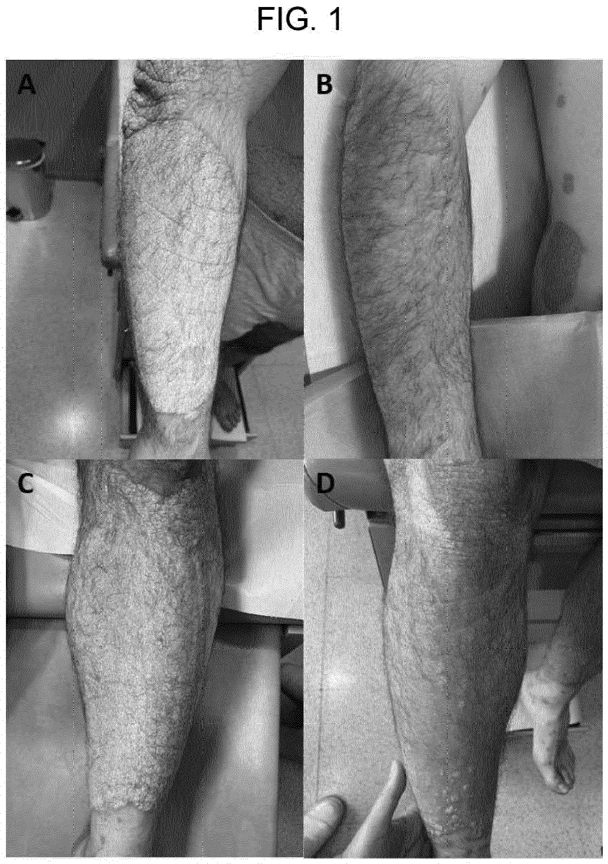

Figure 1 shows the evolution in the affected skin area before (1A, 1C) and after (1B, 1D) treatment with the composition object of the invention.

### Detailed description of the invention

The first aspect of the invention relates to a topical anti-inflammatory composition for use in a treatment of skin inflammation characterised in that it comprises at least one magnesium salt, at least one salt not comprising magnesium, at least one antioxidant, and at least one plant extract, wherein the at least one salt not comprising magnesium is a mixture of sodium chloride, sodium fluoride and potassium chloride. Preferably, the composition of the invention is an aqueous composition, although it may also be formulated as an alcoholic composition. The term "aqueous composition" refers to a composition containing water as the main solvent. In particular, the compositions of the invention comprise at least 95% by weight of water, preferably at least 98% by weight of water in relation to the composition, more preferably 99% by weight of water in relation to the composition. The term "alcoholic composition" relates to a composition containing an alcohol as a solvent, e.g., ethanol. In particular, the alcoholic compositions of the invention comprise at least 10% by weight of alcohol. Unless otherwise indicated, all percentages refer to percentages by weight with respect to the composition.

In a preferred embodiment of the first aspect of the invention, the at least one magnesium salt is selected from organic, inorganic magnesium salts or mixtures thereof. The inorganic magnesium salts are selected from those salts having a high solubility in water, such as halides or sulphates. It is preferably selected from the group of magnesium sulphate, magnesium chloride, magnesium bromide or mixtures thereof. In a preferred embodiment the magnesium salt is magnesium sulphate. The organic magnesium salts are selected from those salts having a high solubility in water, such as, for example, citrates, aspartates or lactates.

In a preferred embodiment of the first aspect of the invention, the composition comprises between 0.01 and 1% by weight with respect to the composition of at least one salt comprising magnesium. Preferably between 0.01 and 0.8% by weight with respect to the composition.

In a preferred embodiment of the first aspect of the invention, the composition comprises between 0.01 and 3% by weight with respect to the composition of at least one salt not comprising magnesium. Preferably between 0.01 and 2.5% by weight with respect to the composition.

In a preferred embodiment of the first aspect of the invention, the composition comprises at least one plant extract selected from solid plant extracts or glycolic plant extracts, or a mixture thereof. The term "glycolic plant extract" refers to the form of botanical extract in which propylene glycol-based solvents are used to obtain the active compounds from plants.

In a preferred embodiment of the first aspect of the invention, the composition comprises between 0.01 and 0.1% by weight in relation to the composition of at least one plant extract. Preferably between 0.01 and 0.05% by weight in relation to the composition.

In a preferred embodiment, the solid plant extract is selected from the group of rosemary dry extract, fermented papaya extract, green tea extract, thyme dry extract, chamomile dry extract, lemon balm dry extract, witch hazel dry extract, ginseng dry extract, ginkgo biloba dry extract and hypericum dry extract or mixtures thereof. The preferred solid plant extract is a mixture of rosemary dry extract, fermented papaya extract, green tea extract.

In a preferred embodiment, the glycolic plant extract is selected from the group of chamomile glycolic extract, oat glycolic extract, sage glycolic extract, centella asiatica glycolic extract, birch glycolic extract, flowering calendula glycolic extract, melilot glycolic extract, white nettle glycolic extract, green nettle glycolic extract and horse chestnut glycolic extract or mixtures thereof. The preferred glycolic plant extract is a chamomile glycolic extract.

In an even more preferred embodiment, the composition comprises rosemary dry extract, fermented papaya extract, green tea extract, and chamomile glycolic extract.

In a preferred embodiment of the first aspect of the invention, the composition comprises at least one antioxidant selected from the group of ferulic acid, nicotinamide, zinc gluconate, melatonin, resveratrol, vitamin C (ascorbic acid), dimethylethanolamine (DMAE), glycolic acid and coenzyme Q, lipoic acid, quercetin, curcumin, ascorbic palmitate, taurine, isoflavones, lycopene, vitamin E, L-carnosine, or mixtures thereof. Preferably, the at least one antioxidant is a mixture of ferulic acid, nicotinamide, zinc gluconate, melatonin, and resveratrol.

Ferulic acid is a natural antioxidant found in various plants, especially in grains such as rice, wheat and oats, as well as in some fruits and vegetables. It is known for its antioxidant and anti-inflammatory properties, making it a valuable component in skin care products and dietary supplements. Helps protect the skin from damage caused by free radicals and contributes to the prevention of premature ageing of the skin
Nicotinamide, also known as vitamin B3 or niacinamide, is a form of water-soluble vitamin B. It plays an essential role in cellular metabolism and has anti-inflammatory properties and the ability to help improve skin tone, reduce the appearance of dark spots and minimise enlarged pores. In addition, it contributes to maintaining the protective barrier of the skin and adequate hydration.

Zinc gluconate is a zinc salt that is frequently used as a nutritional supplement and is also found in skin care products. Zinc is an essential trace element for the functioning of the body, including the maintenance of the immune system and cell regeneration. In skin care, zinc gluconate can help regulate sebum production, reduce inflammation, and promote wound healing.

Melatonin, also known as N-acetyl-5-methoxytryptamine, is a hormone found in higher animals and some algae. In the skin, melatonin plays a crucial role as a free radical scavenger. It increases the activity of enzymes responsible for metabolising free radicals and, in addition, is able to oxidise to eliminate them without producing a redox cycle, which has led it to be called a "suicide antioxidant".

Resveratrol is a polyphenolic compound found in some plants, especially in the skin of red grapes, and also in red wine. It has antioxidant and anti-inflammatory properties that may be beneficial for cardiovascular health and skin. It has been studied for its possible effects in reducing cellular ageing and in protecting against damage caused by free radicals.

By combining the different antioxidants, the synergistic effect of the composition is enhanced, thus achieving a greater benefit for the skin or the body's antioxidant system.

In a preferred embodiment of the first aspect of the invention, the composition comprises between 0.01 and 0.1% by weight relative to the aqueous composition of at least one antioxidant. Preferably between 0.01 and 0.05% by weight in relation to the composition.

In the most preferred embodiment the composition comprises:
- 0.1 and 1% by weight in relation to the magnesium sulphate composition;
- 0.1 and 1% by weight in relation to the composition of sodium chloride, sodium fluoride and potassium chloride;
- 0.01 and 0.1% by weight in relation to the composition of rosemary dry extract, fermented papaya extract, green tea extract and chamomile glycolic extract; and
- 0.01 and 0.1% by weight in relation to the composition of ferulic acid, nicotinamide, zinc gluconate, melatonin, and resveratrol.

The compositions of the examples can be completed with variable amounts of other substances, such as, for example, preservatives, antioxidants, pigments, thickeners, or active ingredients, according to the intended use of the composition.

In other preferred embodiments of the composition, it is in the form of gel, cream, or lotion.

The method of preparing a composition for use in a treatment of skin according to the first aspect comprises the following steps:
a) Providing the necessary amount of solvent;
b) Adding at least one magnesium salt and at least one salt not comprising magnesium to the water from the previous step;
c) Adding at least one antioxidant and at least one plant extract to the mixture of the previous stage.
d) Optionally, adding excipients such as preservatives, antioxidants, pigments, thickeners, or active ingredients, according to the intended use of the composition.

The use of the composition according to the first aspect of the invention is a therapeutic use for the treatment of inflammation in the skin, for example, in the treatment of dermatitis, atopic dermatitis, vitiligo, psoriasis or pruritus.

In a preferred embodiment, the treatment of the affected area of the skin is carried out by immersing the affected area in the composition of the first aspect for a certain period of time.

In another preferred embodiment, the treatment of the affected area of the skin is carried out by spraying the affected area with the composition of the first aspect.

In another preferred embodiment, the treatment of the affected area of the skin is carried out by applying the composition in gel, cream or cream-gel format of the first aspect to the affected area.

In each case and for each patient, the doctor will assess - with the appropriate periodicity - the possible presence and intensity of the pathologies, and will formulate the composition and its application depending on one or the other.

With the compositions of the present invention, inflammation is controlled without the need to abuse substances with side effects, such as antibiotics or corticosteroids, by surprisingly enhancing the effect of the components of the composition of the invention present at low concentrations.

### Examples

### Example 1: Preparation of the aqueous composition of the invention.

The preferred embodiment of the invention is detailed below, by way of non-limiting example

| **Component** | **Concentration (wt %)** |
|---|---|
| Magnesium sulphate | 0.31 |
| Sodium chloride | 0.17 |
| Sodium fluoride | 0.006 |
| Potassium chloride | 0.06 |
| Rosemary dry extract | 0.009 |
| Fermented papaya extract | 0.006 |
| Green tea extract | 0.0006 |
| Ferulic Acid | 0.0006 |
| Nicotinamide | 0.03 |
| Zinc gluconate | 0.009 |
| Melatonin | 0.0000006 |
| Resveratrol | 0.0000009 |
| Chamomile glycolic extract | 0.0000006 |
| Water | Q.S. |

The process for preparing the aqueous composition of the invention comprises the following steps:
a) Providing the necessary amount of water according to the area of the skin to be treated;
b) Adding magnesium sulphate, sodium chloride, sodium fluoride, potassium chloride, ferulic acid, nicotinamide, zinc gluconate, rosemary dry extract, fermented papaya extract, and water-based green tea extract from the previous step;
c) Adding melatonin, resveratrol, and chamomile glycolic extract to the dissolution from the previous stage.

As indicated, the amount of water may vary depending on the area to be treated: if total immersion is required, 100 L (a bathtub) will be prepared, if partial immersion is required, 50 L (a basin), 10 L (a bidet) or 5 L (a basin) of the composition of the invention will be prepared.

In addition to the application of this composition in bathtubs and/or showers, it can also be applied through a vaporiser spray.

### Example 2: Preparation of the composition of the invention - gel, cream or gel-cream.

Other preferred embodiments of the invention are detailed below, by way of non-limiting example. In particular, example 2.1 corresponds to a preparation in cream-gel format, example 2.2 to a preparation in gel format and 2.3 to a composition in cream format

| **Component** | **Example 2.1 (wt %)** | **Example 2.2 (wt %)** | **Example 2.3 (wt %)** |
|---|---|---|---|
| NaCl | 0.336 | 0.336 % | 0.336 % |
| Ferulic Acid | 0.012 | 0.012 | 0.012 |
| NaF | 0.012 | 0.012 | 0.012 |
| KCI | 0.12 | 0.12 | 0.12 |
| Nicotinamide | 0.06 | 0.06 | 0.06 |
| MgSO₄ | 0.6216 | 0.6216 | 0.6216 |
| Papaya dry extract | 0.012 | 0.012 | 0.012 |
| Zn gluconate | 0.018 | 0.018 | 0.018 |
| Rosemary dry extract | 0.018 | 0.018 | 0.018 |
| Green tea dry extract | 0.0012 | 0.0012 | 0.0012 |
| Chamomile glycolic extract | 0.006 | 0.006 | 0.006 |
| Melatonin | 0.004 | 0.004 | 0.004 |
| Resveratrol | 0.004 | | |
| Water-alcohol solution | 0.186 | 0.186 | 0.186 |
| Vitamin C | 1 | - | 1 |
| Vitamin E-acetate | 11 | - | 1 |
| Aqua conservans | 20 | 20 | 20 |
| Cream-gel emulsion | Q.S. | - | - |
| Carboxymethylcellulos e gel | | Q.S. | |
| Oil-in-water emulsion | - | - | Q.S. |

### Example 3: Treatment of skin carried out by applying the composition of the invention.

The composition of the invention, in particular that prepared according to example 1, is applied to patients by total immersion of the patient or the patient's area for at least 20 minutes. The results of the treatment can be seen in Figures 1 and 2 where the before (1A, 1C) and after (1B, 1D) treatment with the compositions of the invention can be observed. As can be seen in the figure, the decrease in inflammation is remarkable.

Alternatively, the compositions of the invention can be applied by spraying on the area to be treated. Other forms of the composition, examples 2.1-2.3, are formulated so that they can be applied directly to the desired area without the need to submerge the affected area. The results obtained are comparable to those obtained with the treatment of the composition of example 1.

## Claims

1. A topical anti-inflammatory composition for use in a treatment of skin inflammation, **characterised in that** it comprises at least one magnesium salt, at least one salt not comprising magnesium, at least one antioxidant, and at least one plant extract, wherein the at least one salt not comprising magnesium is a mixture of sodium chloride, sodium fluoride, and potassium chloride.

2. The composition for use in a treatment of skin inflammation according to claim 1, wherein the at least one salt comprising magnesium is selected from the group of magnesium sulphate, magnesium chloride, magnesium bromide or mixtures thereof.

3. The composition for use in a treatment of skin inflammation according to claim 2, wherein the at least one salt comprising magnesium is magnesium sulphate.

4. The composition for use in a treatment of skin inflammation according to any one of the previous claims, wherein the at least one salt comprising magnesium is in a concentration of 0.1 to 1% by weight in relation to the composition.

5. The composition for use in a treatment of skin inflammation according to claim 1, wherein the at least one salt not comprising magnesium is in a concentration of 0.1 to 3% by weight relative to the composition.

6. The composition for use in a treatment of skin inflammation according to claim 1, wherein the at least one plant extract is a chamomile glycolic extract, oat glycolic extract, sage glycolic extract, centella asiatica glycolic extract, birch glycolic extract, calendula glycolic extract, melilot glycolic extract, white nettle glycolic extract, green nettle glycolic extract, and horse chestnut glycolic extract, or mixtures thereof.

7. The composition for use in a treatment of skin inflammation according to claim 1, wherein the at least one plant extract is a solid extract selected from rosemary dry extract, fermented papaya extract, green tea extract, thyme dry extract, chamomile dry extract, lemon balm dry extract, witch hazel dry extract, ginseng dry extract, ginkgo biloba dry extract, and hypericum dry extract, or mixtures thereof.

8. The composition for use in a treatment of skin inflammation according to claim 1, wherein the at least one plant extract is in a concentration of 0.01 to 0.1% by weight relative to the composition.

9. The composition for use in a treatment of skin inflammation according to claim 1, wherein the at least one antioxidant is selected from the group of ferulic acid, nicotinamide, zinc gluconate, melatonin, resveratrol, vitamin C (ascorbic acid), dimethylethanolamine (DMAE), glycolic acid and coenzyme Q, lipoic acid, quercetin, curcumin, ascorbyl palmitate, taurine, isoflavones, lycopene, vitamin E, L-carnosine, or mixtures thereof.

10. The composition for use in a treatment of skin inflammation according to claim 9, wherein the at least one antioxidant is in a concentration of 0.01 to 0.1% by weight in relation to the composition.

11. The composition for use in a treatment of skin inflammation according to any one of the preceding claims, wherein the composition is in the form of an aqueous composition, alcoholic composition, gel, cream, or lotion.

## Patentansprüche

1. Topische entzündungshemmende Zusammensetzung zur Verwendung bei einer Behandlung von Hautentzündungen, **dadurch gekennzeichnet, dass** sie mindestens ein Magnesiumsalz, mindestens ein Salz, das kein Magnesium umfasst, mindestens ein Antioxidans und mindestens einen Pflanzenextrakt umfasst, wobei das mindestens eine Salz, das kein Magnesium umfasst, eine Mischung aus Natriumchlorid, Natriumfluorid und Kaliumchlorid ist.

2. Zusammensetzung zur Verwendung bei einer Behandlung von Hautentzündungen nach Anspruch 1, wobei das mindestens eine magnesiumhaltige Salz aus der Gruppe Magnesiumsulfat, Magnesiumchlorid, Magnesiumbromid oder Mischungen davon ausgewählt ist.

3. Zusammensetzung zur Verwendung bei einer Behandlung von Hautentzündungen nach Anspruch 2, wobei das mindestens eine magnesiumhaltige Salz Magnesiumsulfat ist.

4. Zusammensetzung zur Verwendung bei einer Behandlung von Hautentzündungen nach einem der vorhergehenden Ansprüche, wobei das mindestens eine magnesiumhaltige Salz in einer Konzentration von 0,1 bis 1 Gew.-%, bezogen auf die Zusammensetzung, vorliegt.

5. Zusammensetzung zur Verwendung bei einer Behandlung von Hautentzündungen nach Anspruch 1, wobei das mindestens eine Salz, das kein Magnesium umfasst, in einer Konzentration von 0,1 bis 3 Gew.-%, bezogen auf die Zusammensetzung, vorliegt.

6. Zusammensetzung zur Verwendung bei einer Behandlung von Hautentzündungen nach Anspruch 1, wobei der mindestens eine Pflanzenextrakt ein Kamillen-Glykolextrakt, Hafer-Glykolextrakt, Salbei-Glykolextrakt, Centellaasiatica-Glykolextrakt, Birken-Glykolextrakt, Calendula-Glykolextrakt, Steinklee-Glykolextrakt, Weißnessel-Glykolextrakt, Grünnessel-Glykolextrakt und Rosskastanien-Glykolextrakt oder Mischungen davon ist.

7. Zusammensetzung zur Verwendung bei einer Behandlung von Hautentzündungen nach Anspruch 1, wobei der mindestens eine Pflanzenextrakt ein Festextrakt ist, der ausgewählt ist aus Rosmarin-Trockenextrakt, fermentiertem Papaya-Extrakt, Grüntee-Extrakt, Thymian-Trockenextrakt, Kamillen-Trockenextrakt, Melissen-Trockenextrakt, Hamamelis-Trockenextrakt, Ginseng-Trockenextrakt, Ginkgo-Biloba-Trockenextrakt und Hypericum-Trockenextrakt oder Mischungen davon.

8. Zusammensetzung zur Verwendung bei einer Behandlung von Hautentzündungen nach Anspruch 1, wobei der mindestens eine Pflanzenextrakt in einer Konzentration von 0,01 bis 0,1 Gew.-%, bezogen auf die Zusammensetzung, vorliegt.

9. Zusammensetzung zur Verwendung bei einer Behandlung von Hautentzündungen nach Anspruch 1, wobei das mindestens eine Antioxidans ausgewählt ist aus der Gruppe Ferulasäure, Nicotinamid, Zinkgluconat, Melatonin, Resveratrol, Vitamin C (Ascorbinsäure), Dimethylethanolamin (DMAE), Glykolsäure und Coenzym Q, Liponsäure, Quercetin, Curcumin, Ascorbylpalmitat, Taurin, Isoflavone, Lycopin, Vitamin E, L-Carnosin oder Mischungen davon.

10. Zusammensetzung zur Verwendung bei einer Behandlung von Hautentzündungen nach Anspruch 9, wobei das mindestens eine Antioxidans in einer Konzentration von 0,01 bis 0,1 Gew.-%, bezogen auf die Zusammensetzung, vorliegt.

11. Zusammensetzung zur Verwendung bei einer Behandlung von Hautentzündungen nach einem der vorhergehenden Ansprüche, wobei die Zusammensetzung in Form einer wässrigen Zusammensetzung, alkoholischen Zusammensetzung, eines Gels, einer Creme oder einer Lotion vorliegt.

## Revendications

1. Composition anti-inflammatoire topique destinée à être utilisée dans un traitement d'une inflammation de la peau, **caractérisée en ce qu'**elle comprend au moins un sel de magnésium, au moins un sel ne comprenant pas de magnésium, au moins un antioxydant, et au moins un extrait végétal, dans laquelle l'au moins un sel ne comprenant pas de magnésium est un mélange de chlorure de sodium, de fluorure de sodium, et de chlorure de potassium.

2. Composition destinée à être utilisée dans un traitement d'une inflammation de la peau selon la revendication 1, dans laquelle l'au moins un sel comprenant du magnésium est choisi dans le groupe du sulfate de magnésium, du chlorure de magnésium, du bromure de magnésium ou de mélanges de ceux-ci.

3. Composition destinée à être utilisée dans un traitement d'une inflammation de la peau selon la revendication 2, dans laquelle l'au moins un sel comprenant du magnésium est le sulfate de magnésium.

4. Composition destinée à être utilisée dans un traitement d'une inflammation de la peau selon l'une quelconque des revendications précédentes, dans laquelle l'au moins un sel comprenant du magnésium est à concentration de 0,1 à 1 % en poids par rapport à la composition.

5. Composition destinée à être utilisée dans un traitement d'une inflammation de la peau selon la revendication 1, dans laquelle l'au moins un sel ne comprenant pas de magnésium est à concentration de 0,1 à 3 % en poids par rapport à la composition.

6. Composition destinée à être utilisée dans un traitement d'une inflammation de la peau selon la revendication 1, dans laquelle l'au moins un extrait végétal est un extrait glycolique de camomille, un extrait glycolique d'avoine, un extrait glycolique de sauge, un extrait glycolique de centella asiatica, un extrait glycolique de bouleau, un extrait glycolique de calendula, un extrait glycolique de mélilot, un extrait glycolique d'ortie blanche, un extrait glycolique d'ortie verte et un extrait glycolique de marron d'Inde, ou des mélanges de ceux-ci.

7. Composition destinée à être utilisée dans un traitement d'une inflammation de la peau selon la revendication 1, dans laquelle l'au moins un extrait végétal est un extrait solide choisi parmi un extrait sec de romarin, un extrait de papaye fermentée, un extrait de thé vert, un extrait sec de thym, un extrait sec de camomille, un extrait sec de mélisse, un extrait sec d'hamamélis, un extrait sec de ginseng, un extrait sec de ginkgo biloba et un extrait sec de millepertuis, ou des mélanges de ceux-ci.

8. Composition destinée à être utilisée dans un traitement d'une inflammation de la peau selon la revendication 1, dans laquelle l'au moins un extrait végétal est à concentration de 0,01 à 0,1 % en poids par rapport à la composition.

9. Composition destinée à être utilisée dans un traitement d'une inflammation de la peau selon la revendication 1, dans laquelle l'au moins un antioxydant est choisi dans le groupe de l'acide férulique, du nicotinamide, du gluconate de zinc, de la mélatonine, du resvératrol, de la vitamine C (acide ascorbique), de la diméthyléthanolamine (DMAE), de l'acide glycolique et de la coenzyme Q, de l'acide lipoïque, de la quercétine, de la curcumine, du palmitate d'ascorbyle, de la taurine, des isoflavones, du lycopène, de la vitamine E, de la L-carnosine, ou de mélanges de ceux-ci.

10. Composition destinée à être utilisée dans un traitement d'une inflammation de la peau selon la revendication 9, dans laquelle l'au moins un antioxydant est à une concentration de 0,01 à 0,1 % en poids par rapport à la composition.

11. Composition destinée à être utilisée dans un traitement d'une inflammation de la peau selon l'une quelconque des revendications précédentes, dans laquelle la composition est sous la forme d'une composition aqueuse, d'une composition alcoolique, d'un gel, d'une crème, ou d'une lotion.
